(19) Europäisches Patentamt / European Patent Office / Office européen des brevets

(11) **EP 4 649 883 A1**

(12) **EUROPEAN PATENT APPLICATION**

(43) Date of publication:
**19.11.2025  Bulletin 2025/47**

(21) Application number: **24175502.4**

(22) Date of filing: **13.05.2024**

(51) International Patent Classification (IPC):
**A61B 3/024** (2006.01)    **A61B 3/032** (2006.01)
**A61B 3/103** (2006.01)

(52) Cooperative Patent Classification (CPC):
**A61B 3/024; A61B 3/032; A61B 3/103**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Hunkeler, Dario Manuel**
**4106 Therwil (CH)**

(72) Inventor: **Hunkeler, Dario Manuel**
**4106 Therwil (CH)**

(74) Representative: **Braunpat AG**
**Peter Merian-Strasse 28**
**4052 Basel (CH)**

(54) **NEW METHOD FOR LOCATING THE BLIND SPOT OF A PERSON AND FURTHER OPHTHALMOLOGICAL METHODS CALIBRATED BY THIS LOCATED BLIND SPOT**

(57)    The present invention is directed to a reliable, precise method for locating the blind spot (200) in the visual field of a person. The distance between the center of a focus point and the center (C) of the located blind spot can then be used to calibrate any ophthalmological test such as e.g. the test of determining the visual acuity of a person, the distance visual acuity as well as the near visual acuity; the test of determining the perimetry of a person, the checking of glaucoma progression, the detection of neurological visual field defects, etc. This allows the self-testing at home by the patient and leads to an early detection of tumors or mute strokes near the visual pathways.

Fig. 5

EP 4 649 883 A1

**Description**

Technical field of the invention

Glaucoma is a group of eye diseases that lead to damage of the optic nerve, which transmits visual information from the eye to the brain. Glaucoma may cause vision loss if left untreated. It has been called the "silent thief of sight" because the loss of vision usually occurs slowly over a long period of time.

There are different types of glaucoma, but the most common are called open-angle glaucoma and closed-angle glaucoma. Inside the eye, a liquid called aqueous humor helps to maintain shape and provides nutrients. The aqueous humor normally drains through the trabecular meshwork. In open-angle glaucoma, the draining is impeded, causing the liquid to accumulate and pressure inside the eye to increase. This elevated pressure can damage the optic nerve.

According to E. A. Lowry et al. ("Comparison of Peristat Online perimetry with the Humphrey Perimetry in a Clinic-Based Setting"), Translational Vision Science & Technology 2016, Vol. 5, No. 4, Article 4 (DOI: 10.1167/tvst.5.4.4), open angle glaucoma will effect over 7 million persons by 2050 only in the US. The detection and management of glaucoma provide unique challenges. Glaucoma detection is hampered by delayed diagnosis due to its prolonged asymptomatic clinical phase. It is estimated that only 34% and 8% of all open angle glaucoma cases are diagnosed in the developed and developing world, respectively. Current guidelines recommend screening for glaucoma with comprehensive eye examinations in highrisk populations. These exams rely on eye care professionals, who are expensive, and only accessible to a small number of patients.

Perimetry is an essential aspect of the comprehensive exam for higher risk patients, with one survey (EY Wong, JE Keeffe, JL Rait, et al. "Detection of undiagnosed glaucoma by eye health professionals." in Ophthalmology. 2004;11 1:1508-1514) showing that more than 90% of undetected glaucoma cases have significant visual field defects that would be apparent with standard perimetry testing such as the Humphrey visual field testing.

Since the typical Humphrey visual field testing is also performed by an eye care professional, it thus, adds more time and cost to the initial evaluation of open angle glaucoma. Therefore, there is a need for a perimetry testing that is cheap and can be carried out by the patient itself.

Prior art

An online, low-cost web-based virtual suprathreshold perimetry test is already known as "Peristat Online Perimetry" ("POP"); see also US 2005/0128434. Peristat online perimetry (POP) sequentially tests a visual field of 24° from fixation horizontally and 20° vertically using four levels of standardized threshold stimuli, i.e. dots of light of a different light intensity, allowing patients to be tested for characteristic visual irregularities in less than 5 minutes per eye. In contrast to Humphrey visual field (HVF), POP is a freely available, online program at the KeepYour-SightFoundation website (available in the public domain at http://www.keepyour-sight.org) that provides unlimited home access, so that the test can be performed by the patient itself at home. A previous pilot study showed POP could be useful when performed in the office with a sensitivity of 84% to 86% and specificity of 94% to 97% compared to HVF for moderate or worse visual field defects. Peristat, however, requires any 17-inch or larger computer monitor, on which the test is then performed. Furthermore, as a single screening test the Peristat would miss at least 14% of moderate or worse glaucomas and up to 46% of early glaucomas.

Rarebit is a similar computer-based visual field testing program which also requires a very large screen, i.e. at least a 15-inch personal computer.

To increase the accuracy of such perimetry tests, it is crucial to take into account the individual blind spot of the patient. Thus, a further development of these self-testings depend on an accurate location of the individual blind spot of a person.

The normal eye can detect stimuli over a 120° range vertically and a nearly 160 degree range horizontally. From the focus point, stimuli can typically be detected 60° superiorly, 70° inferiorly, 60° nasally, and 100 degrees temporally, though the true extent of the visual field depends on several features of the stimulus (size, brightness, motion) as well as the background conditions. The field of vision is often depicted as a three dimensional hill, with the peak sensitivity to stimuli occurring at the focus point under photopic conditions, decreasing rapidly in the 10° around fixation, and then decreasing very gradually for locations further in the periphery. Nerve fibers pass through the sclera at the optic nerve head, typically 10-15° nasal to the focus point. At this location, no photoreceptors are present, creating the so-called blind spot whose exact position is slightly different for each person.

Such a test is e.g. disclosed in paragraph [0067] of US 2022/0192482. This test, however, has to be carried out at a specific testing distance and is not very accurate, since the test does not recognize if the person changes the distance. Only great distance differences are recognized by the test. Furthermore, it does not take into account the size of the display the test is carried out.

Thus, there is a need for a method for locating the individual blind spot of a person that allows self-testing on any display, i.e. also on displays smaller than 15 inch, e.g. on the display of a mobile phone or a tablet, at any testing distance, and that leads to a lower missing rate of neurological visual field defects. In addition such method should lead to a lower missing rate of glaucomas in perimetry tests, especially early glaucomas in perimetry tests calibrated on basis of said located individual blind spot.

Summary of the invention

This need is fulfilled by the present invention that is directed to a method for locating the blind spot in the visual field of a person comprising the following steps:

a) Displaying a focus dot of light (F) that is fixated by one of the person's eyes on a display at a testing distance, whereby the focus dot of light (F) is preferably displayed in the central area of the display;
b) Displaying a movable testing dot of light on the display, whereby the testing dot of light is moving straight from a starting point outside an expected blind spot along an axis towards the center (C) of said expected blind spot,
c) Recording the end position of the testing dot of light that is taken when the person signals that it cannot see the testing dot of light on the display any more;
d) Repeating steps b) and c) a plurality of times, whereby the testing dots of light vary independently from each other with respect to their starting point; and
e) Mapping the located blind spot on basis of the end positions of the testing dots of light.

The present invention is furthermore directed to a method for calibrating an ophthalmological test by using the blind spot located by said method above comprising the following steps:

i) calculating the distance between the focus dot of light and the located blind spot in pixels, and defining the distance between the focus dot of light and the blind spot in degrees;
ii) using the distance calculated in step i) for calibrating all positions or image sizes tested in the ophthalmological test.

Therefore, the present invention is also directed to the use of the located blind spot to calibrate an ophthalmological test, especially the test of determining the visual acuity of a person, the distance visual acuity as well as the near visual acuity; the test of determining the perimetry of a person, the checking of glaucoma progression and/or the test of detecting neurological visual field defects. The test of locating the blind spot is preferably performed under the same room conditions as the ophthalmological test performed thereafter and calibrated by said test of locating the blind spot. That means e.g. since a glaucoma test is performed in darkened rooms, the pre-test for locating the blind spot is also performed in darkened rooms. The perimetry test and the test for neurological visual field defects, however, may also be performed without darkening of the room, but darkening of the room leads to better results.

A further object of the present invention is a method for testing the visual acuity of a person's eye on a display comprising the following steps:

A) Use of the distance between the center of the focus dot of light and the center of the blind spot located by the method according to the present invention described above to calibrate any size of a letter, number, line, symbol, picture or chart tested in said visual acuity test;
B) Optionally displaying a focus dot of light on a display;
C) Displaying different letters, numbers, lines, symbols, pictures or charts with a certain size on the display, whereby their size is calibrated according to step A), and recording for every displayed letter, number, line, symbol, picture or chart the input of the person;
D) Repeating step C) a plurality of times, whereby the letters, numbers, lines, symbols, pictures or charts have a different size than the letters, numbers, lines, symbols, pictures or charts already displayed;
E) Displaying a reference dot of light within the area of the located blind spot of the person on the display and optionally repeating steps B) to D), depending on the person's input.

Detailed description of the invention

The method for locating the blind spot in the visual field of a person according to the present invention is now described in more detail below. One eye is tested by said method at a time. The untested eye is covered and the patient gazes with the other eye at the focus dot of light. The focus dot of light which may also called "focus point" is fixated by the person's eye to be tested the whole time when steps b) to d) are performed. The testing distance has to be kept constant during the whole measurement.
For the right eye the physiological blind spot is right from the focus point; for the left eye the physiological blind spot is left from the focus point.

The focus dot of light is displayed in the central area of the display. The "central area" can be the middle area of the display, but does not necessarily have to be the center of the display. One could devide the display in three equally largy areas vertically and horizontally, i.e. in total in nine equally large areas. "Middle area of the display" means the area that is located **either** in the second third vertically **or** in the second third horizontally, preferably the area that is located in the second third vertically **and** in the second third horizontally.

The focus dot of light is preferably circular. It can, however, also have any other form such as e.g. being triangular, rectangular, pentagonal, hexagonal, star-shaped, or have the form of a square or a cross. The size of the focus dot of light may be adjusted to the size of the display. It is, however, also possible to use a focus dot of light with a standardized size in the beginning, and as soon as the blind spot is located, also scale the size of the focus dot of light accordingly in the following test.

In an embodiment of the method according to the present invention the dot of lights are white or of a bright colour such as bright blue, bright green or bright red, just to name a few, non-limiting colours, and the contrasting background is black. If the contrasting background is white, the dot of light may be a dark color such as dark blue, dark green or dark red, just to name a few, non-limiting colours, or black. The intensity of the dot of light can vary within the standard dynamic range. This is especially the case, if the presence of glaucoma is tested: Patients with glaucoma become continuously blind in their visual field. The sensitivity is slowly declining, i.e. less intense dot of lights cannot be seen any more; a dot of light with an intensity of 30% may e.g. not be seen any more, whereas a dot of light with an intensity of 40% can still be seen. Therefore, it is crucial to test dots of light with different light intensity in this test for glaucoma. The unity for the brightness, i.e. the light intensity, is "NIT" or "dB".

The Humphrey perimeter tests light intensities over five orders of magnitudes, from 10,000 apostilbs (asb) to 0.1 asb. Every log order change in light intensity corresponds to 10 dB, such that the machine can measure sensitivities over a 50 dB range. Test locations unable to see a stimulus of 10,000 asb are assumed to be totally blind (though they may still see brighter lights (or other visual stimuli such as motion)), and are assigned a value of 0 dB. The threshold values reported for each location reflect the extent to which light can be dimmed and still detected. For example, a value of 30 dB indicates that the stimulus can be dimmed 1000-fold to 10 asb.

The method according to the present invention can be carried out on any display, especially on any rectangular display with a diagonal in the range of 4 inch (10.16 cm) to 100 inch (2.54 m), especially of 6 inch (15,24 cm) to 55 inch (139.7 cm). Displays, especially rectangular ones, with a diagonal in the range of 11 inch (27.94 cm) to 32 inch (81.28 cm) are especially comfortable for the person to be tested. Thus, the method is preferably carried out on any display with a diagonal in the range of 11 inch (27.94 cm) to 32 inch (81.28 cm). The display may be the display of a mobile phone, a tablet, a laptop, a computer or a TV, most preferably it is the display of a computer, a TV or a portable device such as a tablet or a laptop.

The pixel density of the display of the device is not relevant for the method according to the present invention. Preferably the pixel density of the display of the device that is used for performing the method for locating the blind spot according to the present invention is preferably in the range of 50 to 5000 pixel/inch, more preferably in the range of 100 to 2000 pixel/inch, even more preferably in the range of 200 to 1000 pixel/inch, most preferably in the range of 300 to 700 pixel/inch.

The method according to the present invention can be carried out at any testing distance between the display and the person's eye. Preferably a testing distance is chosen so that the person performing the test has a comfortable position to the display. The general rule is that the bigger the display is, the bigger is the testing distance. The testing distance is preferably in the range of 10 to 15 cm for mobile phones, in the range of 20 to 45 cm for tablets, in the range of 30 cm to 2 m for computers and in the range of 50 cm to 5 m for big TVs. These numbers are given as example only and are in no way considered as being limited.

When the person is closer to the screen, the angle examined during perimetry is increased. This makes variable testing possible. Thus, the test can tell the patient to move closer to the screen for a larger test range.

In a preferred embodiment of the method according to the present invention, the starting points of the testing dots of light are located independently from each other at various distances and/or at various angles relative to the center (C) of the expected blind spot. The velocity the testing dot of light is moving with is preferably in the range of 1 to 100 pixel/sec, more preferably in the range of 5 to 50 pixel/sec.

In a further preferred embodiment of the method according to the present invention, the expected blind spot is located in an area being 10° to 20° lateral and -2° to 5° below the focus dot of light, preferably the expected blind spot is located in an area being 12° to 18° lateral and -3° to 5° below the focus dot of light.

In another preferred embodiment of the method according to the present invention, the starting points of the first and second testing dots of light are located on a first straight line, whereby said first straight line is parallel to a reference straight line and below said reference straight line, preferably 1° to 2° below. Said reference straight line is a horizontal line going through the center of the focus dot of light (F) and being 1° to 2° above the center (C) of the expected blind spot. Hereby the starting point of one of the two testing dots of light is located on said first straight line near the focus dot of light (F), and additionally in the area between the center of the focus dot of light (F) and the center (C) of the expected blind spot; and the starting point of the other testing dot of light is located beyond the center (C) of the expected blind spot, preferably near the edge of the display. The starting points of these two testing dots of light can be axially symmetrical to each other.

In a preferred embodiment of the method of the present invention the first testing dot of light is located on said first straight line near the focus dot of light (F), and additionally in the area between the center of the focus dot of light (F) and the center (C) of the expected blind spot; and the starting point of the second testing dot of light is located beyond the center (C) of the expected blind spot, preferably near the edge of the display.

In an also preferred embodiment of the method according to the present invention, the starting points of the third and forth testing dots of light are located on a second straight line through the center of the expected blind spot or through the middle of the line connecting the two end points of the first and second dot of light, whereby the second straight line is rectangular to the first straight line. Hereby one of the starting points of the third and forth testing dots of light is located on the second straight line above the expected blind spot and the other below the expected blind spot. The starting points of the third and forth testing dots of light can be axially symmetrical to each other.

In a preferred embodiment testing dots of light are used whose starting positions are on a diagonal. It is especially advantageous to have additional dots of light starting on positions on a straight line that has an angle in the range of 10° to 80° with the first straight line **or** the second straight line, preferably in the range of 20° to 70° with the first straight line **or** the second straight line, more preferably in the range of 30° to 60° with the first straight line **or** the second straight line, most preferably in the range of 40° to 50° with the first straight line **or** the second straight line.

It is furthermore especially advantageous to have additional dots of light starting on positions on a third straight line that has an angle in the range of 10° to 80° with the first straight line **and** additional dots of light starting on positions on a forth straight line that has an angle in the range of 10° to 80° with the second straight line, preferably additional dots of light starting on positions on a third straight line that has an angle in the range of 20° to 70° with the first straight line **and** additional dots of light starting on positions on a forth straight line that has an angle in the range of 20° to 70° with the second straight line, more preferably additional dots of light starting on positions on a third straight line that has an angle in the range of 30° to 60° with the first straight line **and** additional dots of light starting on positions on a forth straight line that has an angle in the range of 30° to 60° with the second straight line, most preferably additional dots of light starting on positions on a third straight line that has an angle in the range of 40° to 50° with the first straight line **and** additional dots of light starting on positions on a forth straight line that has an angle in the range of 40° to 50° with the second straight line.

Using two testing dots of light on the same axis in opposite directions has the advantage, that delays of the person's input due to the reaction time do not play a role.

Preferably at least two end points of said testing dots of light are recorded, more preferably four end points of said testing dots of light are recorded, most preferably four to eight end points of said testing dots of light are recorded. The more end points of said testing dots of light are recorded, the more accurate is the location of the blind spot. Therefore, of course also more than eight end points of said testing dots of light may be recorded.

The center of all end points is the center of the located blind spot. The result is defined as an angle or is calculated with the measurement of the distance between the eye and focus point, and indicates the position of the blind spot in relation to the focus dot of light.

In another preferred embodiment of the method according to the present invention, the method further comprises the step of:

f) Displaying a reference dot of light within the located blind spot on the display and optionally repeating steps b) to e), depending on the person's input.

If the person can see the reference dot of light, that means that the method of locating the blind spot was not properly carried out, e.g. because the person changed its position and thus, the testing distance, and therefore the method of locating the blind spot has to be repeated, or the position of the person has to be changed to a position, where the reference dot of light cannot be seen by the person on the display.

If the person cannot see the reference dot of light on the display, that means that the location of the blind spot was correct. Then steps b) to e) do not have to be repeated.

In any way, the person has to keep a position, where the reference dot of light cannot be seen on the display for performing further tests.

The signal of the person in step c) and the input of the person in step f) can independently from each be set by tactile means or verbally. "Tactile" means that any key of the device can be hit or the display be touched. "Verbally" means by voice command.

The accuracy of the above described method according to the present invention is even further improved, if the method further comprises the step of recording the testing distance between the display and the person's eye and optionally retrieving the display's properties. One of the display's properties is e.g. the pixel density. With the known pixel density, the distance x between between the center C of the located blind spot and the center of the focus dot of light F can be calculated in cm or inches. The exact angle $\alpha$ between the center C of the located blind spot and the center of the focus dot of light F can then be calculated with the following equation:

$$\alpha = \tan^{-1}\left(\frac{x}{l}\right)$$

whereby l is the testing distance between the patient's eye and the display in cm.

If the blind spot and its center have been located successfully by the method of the present invention, they can be used to calibrate any opthalmological test. Therefore, the present invention is also directed to a method for calibrating an ophthalmological test by using the blind spot located by the method accroding to the present invention comprising the following steps:

i) calculating the distance between the focus dot of light and the center of the located blind spot in pixels, and defining the distance between the focus dot of light and the blind spot in degrees;
ii) using the distance calculated in step i) for calibrating all positions z or image sizes z to be tested on the display in the ophthalmological test with the following equation:

$$z = x \; \frac{\tan(y \; in \; [°])}{\tan(center \; of \; the \; located \; blind \; spot \; in \; [°])}$$

whereby x is the distance of the center of the located blind spot to the focus dot of light in pixels and y is the angle in degrees to be tested.

Surprisingly, the inventors found out, that there is an alternative method to to calibrate any opthalmological test. This method is based on the evaluation that for most people the blind spot is located in an area 12° to 18° lateral to the vision field, preferably in an area 13° to 17° lateral to the vision field, more preferably in an area 14° to 16° lateral to the vision field, most preferably in an area 15° lateral to the vision field, **and** in an area of 3° above to 5° below the vision field, preferably in an area of 2° above to 4° below the vision field, more preferably in an area of 0° to 2° below the vision field, most preferably in an area of 1° to 2° below the vision field. Thus, the above calculations are based on these values for the blind spot. The values given for the lateral and the horizontal position of the blind spot and their preferences also encompass any combination thereof.

The calibration of any ophthalmological test allows the use of any display to carry out these ophthalmological tests. This allows the self-testing at home and is especially beneficial in cases where multiple checks are necessary. Non-limiting examples of ophthalmological tests that can be calibrated accordingly are the test of determining the visual acuity of a person, the distance visual acuity as well as the near visual acuity; the test of determining the perimetry of a person, the checking of glaucoma progression and the detection of neurological visual field defects.

Non-limiting examples of neurological visual field defects are besides glaucoma e.g. hemianopsia and quadrant anopsia caused by strokes, brain ischemia, transient ischemic attacks, cerebral hemorrhages, pituitary tumors or brain tumors.

Thus, the present invention is also directed to a method for testing the visual acuity of a person's eye on a display. Testing the visual acuity implies determining the smallest letters you can read on the display. For persons that cannot read, especially children, numbers, lines, symbols or pictures are used. The symbols may have direction-specific features as e.g. and especially the Landolt C symbols and the corresponding "E" symbols. The test is done, one at a time for each eye, whereby the other eye is covered. Advantageously the method for locating the blind spot (= "pre-test") is carried out for the right eye and then the visual acuity test with the same eye, and then the same is done with the left eye: first the pre-test and then the visual acuity test.

From a scientific point of view, the smallest angle that can still be seen (in angular minutes) is measured in the visual acuity test, because visual acuity refers to the ability of the retina to perceive two points as separate. In other words, the visual acuity test measures how small the angle between two points can be. A visual acuity of 1.0 would be 100% visual acuity compared to the general population.

The method for testing the visual acuity of a person's eye on a display comprises the following steps:

A) Use of the distance between the center of the focus dot of light and the center of the blind spot located by the method according to the present invention described above to calibrate any size of a letter, number, line, symbol, picture or chart tested in said visual acuity test;
B) Optionally displaying a focus dot of light on the display;
C) Displaying different letters, numbers, lines, symbols or pictures with a certain size on the display, whereby their size is calibrated according to step A), and recording for every displayed letter, number, line, symbol, picture or chart the input of the person;
D) Repeating step C) a plurality of times, whereby the letters, numbers, lines, symbols, pictures or charts have a different size than the letters, numbers, lines, symbols, pictures or charts already displayed;

E) Displaying a reference dot of light within the area of the located blind spot of the person on the display and optionally repeating steps B) to D), depending on the person's input.

Charts are e.g. a sequence of defined letters.

The aim of this test is to find out the size of the letters, numbers, lines, symbols, pictures or charts the patient can still recognize correctly. Thus, the test usually starts with a big size of the shown letters, numbers, lines, symbols, pictures or charts, and continuously shows letters, numbers, lines, symbols, pictures or charts that get smaller and smaller in size. Depending on the determined acuity glasses or contact lenses are needed to compensate for the loss of acuity.

The same display as used for the above described method for locating the blind spot according to the present invention is also used for the visual acuity test. That means that as soon as the blind spot of the individual person is located (first test, pre-test), further tests may be performed on the same display while calibrating each with the outcome of the first test.

The pre-test to locate the blind spot can also be used to calibrate any perimetry test. In one perimetry test e.g. dots of light up to 30° are displayed in 2° steps. To generate a dot of light in the perimetry test at a certain position, the position z [pixels] can be calculated with the following equation:

$$z = x \; \frac{\tan(y \; in \; [°])}{\tan(center \; of \; the \; located \; blind \; spot \; in \; [°])}$$

whereby x is the distance of the center of the located blind spot to the focus dot of light in pixels and y is the position to be tested [°].

Thus, the perimetry test can be adjusted to the size of any display, and thus, be performed at any display. The position of the blind spot also allows to recognize which positions [°] may be tested and which not. If e.g. a greater angle should be tested, the test could signal the person to move closer to the display.

The aim of this perimetry test is to show the patient multiple dots of light at different positions and to map whether these dots of lights are seen by the patient. Areas where no dots of lights are recorded mean that there are neurological visual field impairements, a finding that should initiate the search for their causes.

Therefore, a further object of the present invention is a method for testing the perimetry of a person to detect neurological visual impairments comprising the following steps:

- Use of the distance between the center of the focus dot of light and the center of the blind spot located by the method of the present invention as disclosed above to calibrate the positions of testing dots of light;
- Optionally displaying a focus dot of light on a display;
- Displaying a testing dot of light at a certain position on the display and recording the input of the person for the displayed dot of light;
- Repeating the former step a plurality of times, whereby all testing dots of light differ from each other with regard to their position on the display;
- Displaying a reference dot of light within the area of the located blind spot of the person on the display and optionally repeating the former steps, depending on the person's input;
- Mapping the perimetry of the person based on the input recorded.

The perimetry of a person can also be used as an indicator for the position of lesions of the *nervus opticus,* the *tractus opticus* and the *radiatio optica.*

The perimetry test can also be used for glaucoma detection. In this case the pre-test to locate the blind spot is carried out in a darkened room. The following perimetry test is then also carried out under the same room conditions, i.e. also in a darkened room. Here not only dots of lights at different positions are shown, but their brightness/light intensity is also modified.

Thus, the present invention is also directed to a method for testing the perimetry of a person to detect a glaucoma comprising the following steps:

- Use of the distance between the focus dot of light and the blind spot located by the method of the present invention as disclosed above to calibrate the positions of testing dots of light;
- Displaying a focus dot of light on a display;
- Displaying a testing dot of light at a certain position on the display and recording the input of the person for the displayed dot of light;
- Repeating the former step a plurality of times, whereby all testing dots of light differ from each other with regard to their position on the display, and whereby each position of a dot of light has to be tested with different brightnesses of the dot of light on the display;

- Displaying a reference dot of light within the area of the located blind spot of the person on the display and optionally repeating the former steps, depending on the person's input;
- Mapping the perimetry, being dependent on the brightness of the testing dots of lights, of the person based on the input recorded.

The methods for testing the perimetry of a person according to the present invention could be called "**c**omputer-**a**ssisted **s**elf **a**ssessment perimetry" ("C-ASAP").

C-ASAP differs from the tests of other providers in that there is no restriction with respect to usable devices, no restriction with respect to display technology (pixel density) and no predetermined distance to the display.

Further advantages of the methods of the present invention are that it is no longer necessary to perform such tests by an eye health practitioner. The methods/tests according to the present invention can be carried out by nursing staff and general practitioners, as well as by the persons/patients themselves. This is especially valuable for a precise visual field measurement to detect neurological visual field impairments much earlier and assess visual acuity with regard to the ability to drive a car. Patients with prolonged episodes of headache are often treated for migraine, so tumors or mute strokes near the visual pathways are not detected early. The neurological visual field defects are often undetected, because the brain or other healthy eye compensate for the blind spot of the one eye. Patients being at home can, nevertheless, get a reliable diagnosis for neurological visual field defects with the above disclosed methods, either alone or in cooperation with their usual general practitioner. This allows a faster medical care for these patients.

Glaucoma patients can carry out follow-up checks independently at home and much more often.

Since the invention helps detecting tumors in the visual pathways much earlier, cost-intensive therapies are avoided and a second stroke in silent stroke patients prevented through early and efficient therapy. That means in the long run that disability-adjusted life ("DALYs ") years can be shortened or prevented.

Short description of the drawings

Fig. 1 shows the display as shown to the patient with the focus dot of light, the first testing dot of light and the direction of its move, as well as the expected blind spot of the right eye.

Fig. 2 shows the display as shown to the patient with the focus dot of light, the first testing dot of light and the direction of its move, as well as the expected blind spot of the left eye.

Fig. 3 shows the move of the second, third and fifth testing dot of light.

Fig. 4 shows the distance between the focus dot of light and the located blind spot.

Fig. 5 and 6 illustrate the calibration of the perimetry test.

Fig. 7 and 8 illustrate the calibration of the visual acuity test.

Detailed description of the drawings

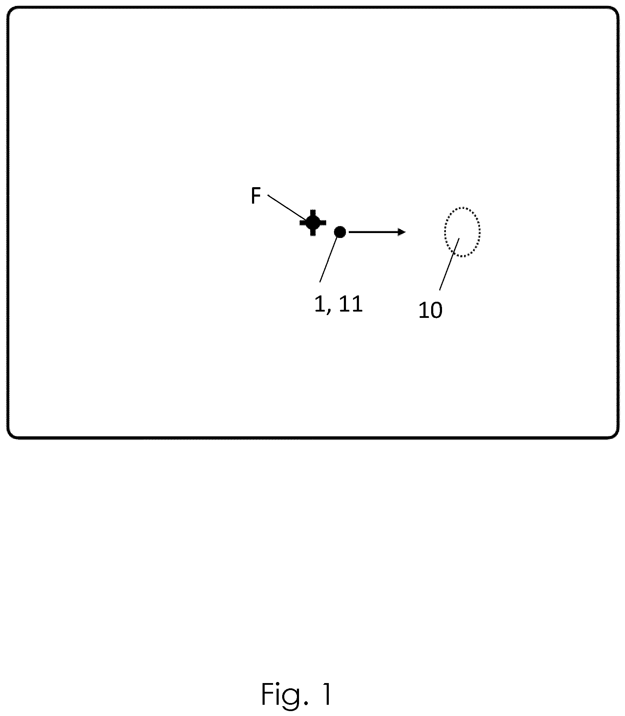

Fig. 1 shows the display as shown to the patient when the right eye is tested. The method according to the present invention to locate the blind spot of the right eye starts with displaying the focus dot of light F, the patient has to fixate, on the display. While fixating the focus dot of light F, the first testing dot of light 1 starts moving from its starting position 11 on a first straight horizontal line to the expected blind spot 10 of the right eye. This first straight line is located some degrees below a horizontal line through the center of the focus dot of light F.

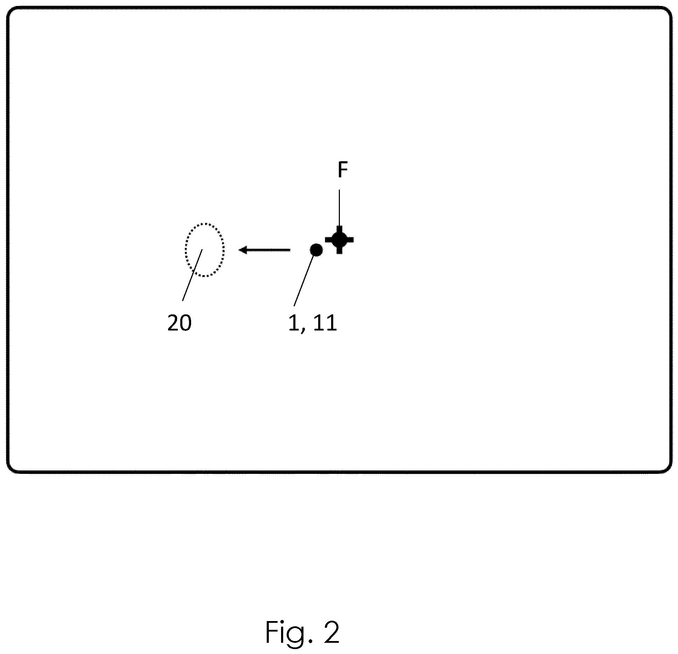

Fig. 2 shows the display as shown to the patient when the left eye is tested. The method according to the present invention to locate the blind spot of the left eye starts with displaying the focus dot of light F , the patient has to fixate, on the display. While fixating the focus dot of light F, the first testing dot of light 1 starts moving from its starting position 11 on a first straight horizontal line towards the expected blind spot 20 of the left eye. This first straight line is located parallel and some degrees below a straight horizontal line through the center of the focus dot of light towards the center of the expected blind spot 20.

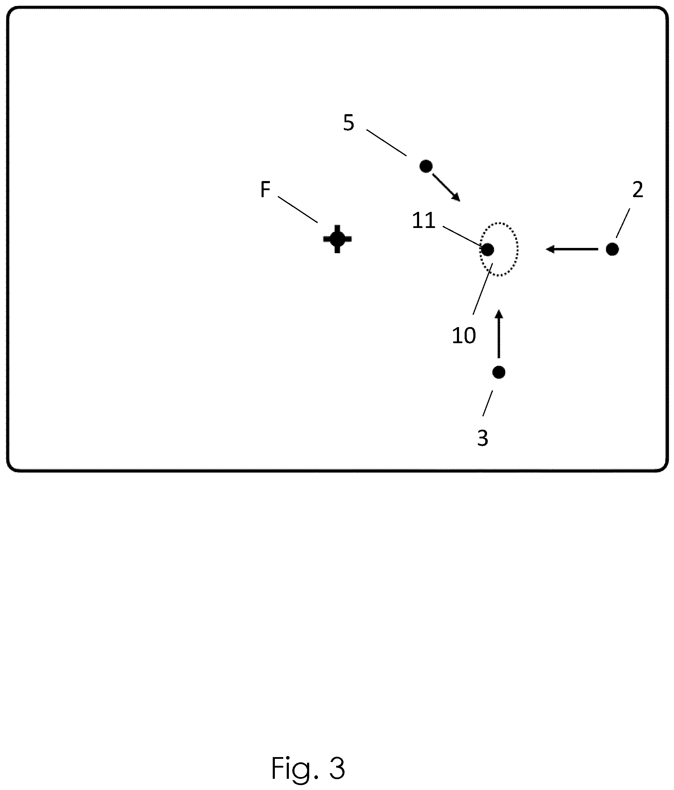

Fig. 3 shows the move of the second, third and fifth testing dot of light 2, 3, 5 starting from their starting positions 12, 13, 15, whereby the end position 11 of the first testing dot of light has already been recorded.

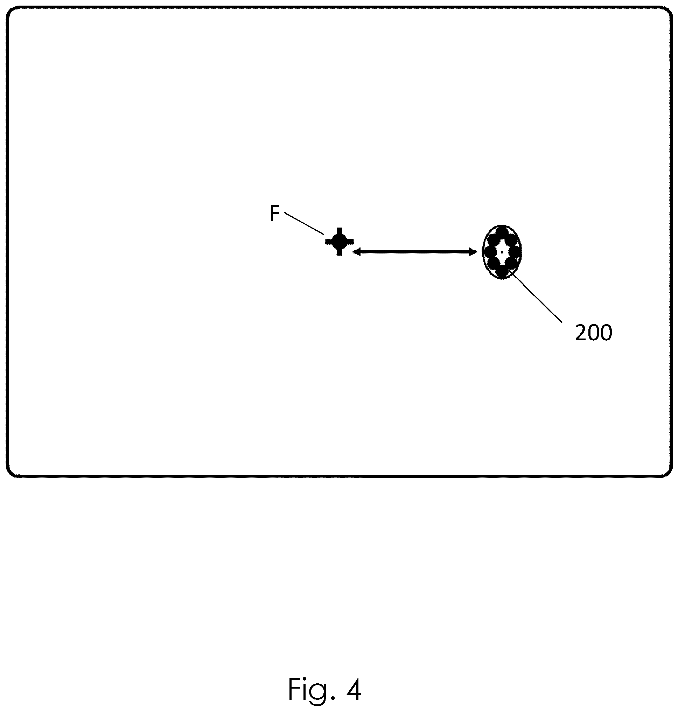

Fig. 4 shows the distance between the focus dot of light F and the mapped, located blind spot 200 based on the end points of eight testing dots of light. This distance is used to calibrate positions and image sizes in any ophthalmological test. To check whether the patient kept its testing distance a reference dot of light may be shown within the area of the located blind spot 200. If the reference dot of light is seen, the position of the patient has changed, and either has to be corrected or the test repeated.

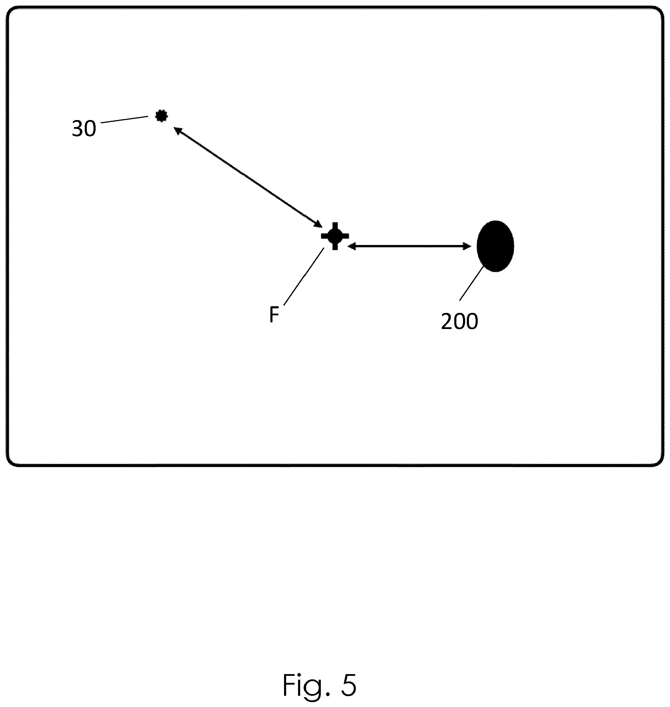

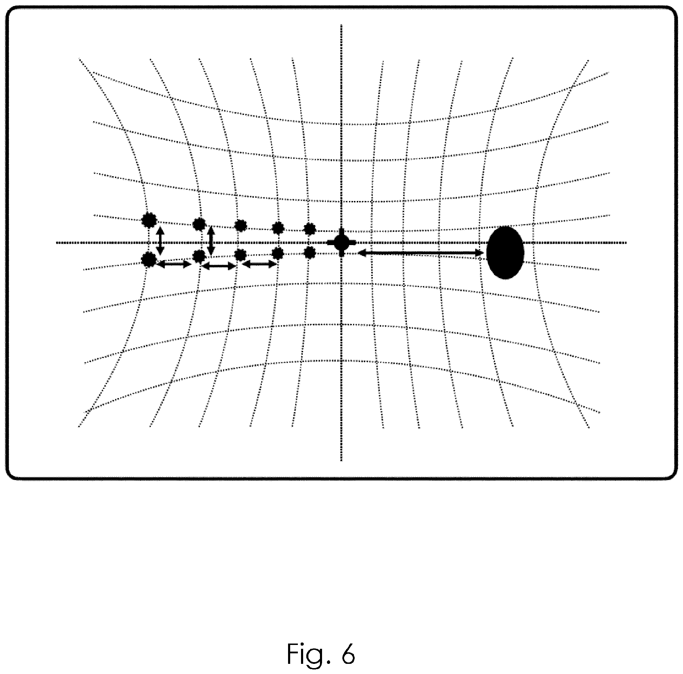

Fig. 5 and 6 illustrate the calibration of the perimetry test. Fig. 5 shows an exemplary testing dot of light 30, whose position can be scaled based on the distance between the focus dot of light F and the mapped, located blind spot 200. The visual field to be tested in the perimetry test is shown in Fig. 6. The field has nearly horizontal and nearly vertical lines. Their bending is due to the fact that the human retina is nearly a sphere and when projected in angles on a flat ground/display it will be warped. The longitudinals and latitudinals are called in degrees. As shown in Fig. 6 every longitudinal and every lateral distance can be calibrated with the distance between the focus dot of light F and the mapped, located blind spot 200.

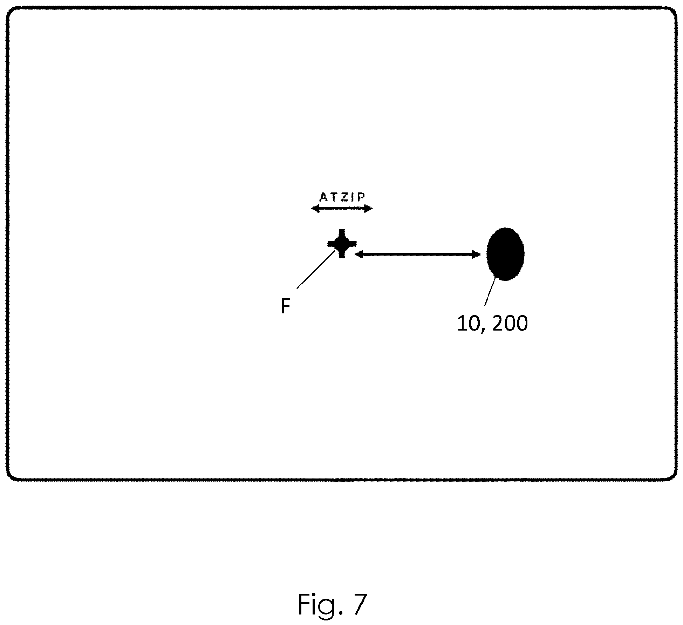

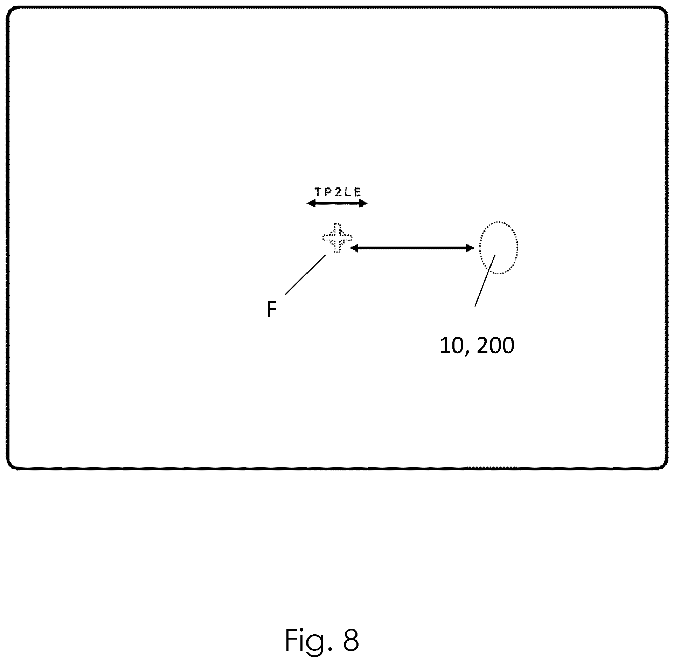

Fig. 7 and 8 illustrate the calibration of the visual acuity test. The size of the charts "ATZIP" and "TP2LE" (so-called Snellen charts) is calibrated with the distance between the focus dot of light F and the mapped, located blind spot 200 or the defined/expected blind spot 10. In the visual acuity test the focus dot of light F can be shown in the beginning to direct the person's focus to this point. During the testing the focus dot of light is not shown any more or only from time to time (indicated with the dotted lines in Fig. 8). To make sure that the chosen testing distance is kept during the test, a reference dot of light can be shown in the area of the blind spot. As long as this reference dot of light is unseen by the patient, if the patient focuses on the focus dot of light, the testing distance is kept. Seeing this reference dot of light, however, means that the patient has changed its position. Then the test has to be repeated or the position changed until the reference dot of light is not seen any more.

The present invention is now further illustrated by the following non-limiting examples.

Examples

Example 1: Calibration of a perimetry test

The blind spot of a person is either defined as being located 15° lateral to the focus dot of light, or it is located with the method according to the present invention. Hereby four to eight end points are recorded. The first, second, third and forth testing dot of light are moved as disclosed above. The fifth and sixth testing dot of light are moved along a straight line, whereby the angle between said straight line and the first straight line is 45°. The seventh and eighth testing dot of light are moved along a straight line, whereby the angle between said straight line and the second straight line is 45°. The outcome of the test is that the blind spot is located at 15° lateral to the focus point. The distance x between the center C of the blind spot and the center of the focus dot of light F is determined in pixels and defined in °.

Furthermore, preferably the distance between the eye of the person and the display is also measured and put into the test. Thereby, the located blind spot can be calculated to be at an exact angle, not at a previously defined angle such as most preferably at 15°.

This allows now to calculate every position z on the display in pixles by using its angle y with the following equation:

$$z = x\ \frac{\tan(y\ in\ [°])}{\tan(15°)}$$

The center C of the blind spot is e.g. located 500 pixels (x) laterally from the focus point.

In the perimetry test dots of light up to 30° are displayed in 2° steps. To generate a point in the perimetry test at a 6° position, the point has to be shown at a position 196 pixels away from the focus point.

$$z = 500\ \frac{\tan(6)}{\tan(15)} = 196$$

Thus, the perimetry test can be adjusted to the size of any display, and thus, be performed at any display. This is advantageous compared to the Peristat test, since in the Peristat test the person has to adjust its distance to the display. This is the cause of errors since it cannot be assured that the person is centrally placed for the test, which leads to the test results not being accurate.

List of reference signs

| F | focus dot of light |
|---|---|
| 1, 2, 3, 4, 5, ... | testing dot of light |
| 11, 12, 13, 14, 15, ... | starting point of testing dot of light |
| 21, 22, 23, 24, 25, ... | end point of testing dot of light |

| 10 | expected blind spot of the right eye |
|---|---|
| 20 | expected blind spot of the left eye |
| C | center of expected/located blind spot |

30     testing dot of light in a perimetry test

| 200 | located blind spot |
|---|---|
| x | distance between the center C of the blind spot and the center of the focus dot of light F |

**Claims**

1. A method for locating the blind spot (200) in the visual field of a person comprising the following steps:

   a) Displaying a focus dot of light (F) that is fixated by one of the person's eyes on a display at a testing distance, whereby the focus dot of light (F) is preferably displayed in the central area of the display;
   b) Displaying a movable testing dot of light (1) on the display, whereby the testing dot of light (1) is moving straight from a starting point (11) outside an expected blind spot (10) along an axis towards the center (C) of said expected blind spot (10),
   c) Recording the end position (21) of the testing dot of light that is taken when the person signals that it cannot see the testing dot of light (1) on the display any more;
   d) Repeating steps b) and c) a plurality of times, whereby the testing dots of light (1, 2, 3, 4, ...) vary independently from each other with respect to their starting point (11, 12, 13, 14, ...); and
   e) Mapping the located blind spot (200) on basis of the end positions (21, 22, 23, 24, ...) of the testing dots of light.

2. The method according to claim 1, **characterized in that** the method can be carried out on any display, preferably on any display with a size in the range of 4 inch (10,16 cm) to 100 inch (2,54 m) and/or a pixel density in the range of 50 to 5000 pixel/inch, more preferably on the display of a mobile phone, a tablet, a laptop, a computer or a TV; **and/or characterized in that** the method can be carried out at any testing distance between the display and the person's eye, preferably at any testing distance in the range of 10 cm to 5 m.

3. The method according to claim 1 or claim 2, **characterized in that** the starting points (11, 12, 13, 14, ...) of the testing dots of light are located independently from each other at various distances and/or at various angles relative to the center (C) of the expected blind spot; **and/or characterized in that** at least two end points of said testing dots of light are recorded, preferably four end points of said testing dots of light are recorded, more preferably four to eight end points of said testing dots of light are recorded.

4. The method according to any one of claims 1 to 3, **characterized in that** the expected blind spot is located in an area being 10° to 20° lateral and -2° to 5° below to the focus dot of light, preferably the expected blind spot is located in an area being 12° to 18° lateral and - 3° to 5° below to the focus dot of light.

5. The method according to any one of claims 1 to 4, **characterized in that** the starting points (11, 12) of the first and second testing dots of light are located on a first straight line, whereby said first straight line is parallel to a reference straight line and below said reference straight line, and said reference straight line goes through the center of the focus dot of light (F) and is above the center (C) of the expected blind spot; whereby the starting point of one of the two testing dots of light (1, 2) is located on said first straight line near the focus dot of light (F), and additionally in the area between the center of the focus dot of light (F) and the center (C) of the expected blind spot; and whereby the starting point of the other testing dot of light is located beyond the center (C) of the expected blind spot, preferably near the edge of the display.

6.  The method according to any one of claims 1 to 5, **characterized in that** the starting points (13, 14) of the third and forth testing dots of light are located on a second straight line through the center of the expected blind spot (10) or through the middle of the line connecting the two end points (23, 24) of the first and second dot of light, whereby the second straight line is rectangular to the first straight line; and whereby one of the starting points (13, 14) of the third and forth testing dots of light is located on the second straight line above the expected blind spot and the other below the expected blind spot.

7.  The method according to any one of claims 1 to 6, further comprising the step of:
    f) Displaying a reference dot of light (201) within the located blind spot (200) on the display, and optionally repeating steps b) to e), depending on the person's input.

8.  The method according to any one of claims 1 to 7, **characterized in that** the signal of the person in step c) is set by tactile means or verbally.

9.  The method according to any one of claims 1 to 8, further comprising the step of recording the testing distance between the display and the person's eye and optionally retrieving the display's properties.

10. A Method for calibrating an ophthalmological test by using the blind spot located by the method of any one of claims 1 to 9 comprising the following steps:

    i) calculating the distance between the focus dot of light (F) and the located blind spot (200) in pixels, and defining the distance between the focus dot of light (F) and the blind spot (200,10) in degrees;
    ii) using the distance calculated in step i) for calibrating all positions or image sizes tested in the ophthalmological test.

11. Use of the blind spot located by the method according to any one of claims 1 to 9 to calibrate an ophthalmological test.

12. The method according to claim 10 or the use according to claim 11, whereby the ophthalmological test is selected from the test of determining the visual acuity of a person, the test of determining the perimetry of a person, the checking of glaucoma progression and the detection of neurological visual field defects.

13. A method for testing the visual acuity of a person comprising the following steps:

    A) Use of the distance between the center of the focus dot of light (F) and the center of the blind spot (200) located by the method of any one of claims 1 to 9 to calibrate any size of a letter, number, line, symbol, picture or chart tested in said visual acuity test;
    B) Optionally displaying a focus dot of light (F) on a display;
    C) Displaying different letters numbers, lines, symbols, pictures or charts with a certain size on the display, whereby their size is calibrated according to step A), and recording for every displayed letter, number, line, symbol, picture or chart the input of the person;
    D) Repeating step C) a plurality of times, whereby the letters, numbers, lines, symbols, pictures or charts have a different size than the letters, numbers, lines, symbols, pictures or charts already displayed;
    E) Displaying a reference dot of light within the area of the located blind spot of the person on the display, and optionally repeating steps B) to D), depending on the person's input.

14. A method for testing the perimetry of a person to detect neurological visual impairments comprising the following steps:

    - Use of the distance between the center of the focus dot of light (F) and the center of the blind spot (200) located by the method of any one of claims 1 to 9 to calibrate the positions of testing dots of light;
    - Optionally displaying a focus dot of light (F) on a display;
    - Displaying a testing dot of light at a certain position on the display and recording the input of the person for the displayed dot of light;
    - Repeating the former step a plurality of times, whereby all testing dots of light differ from each other with regard to their position on the display;
    - Displaying a reference dot of light within the area of the located blind spot of the person on the display and optionally repeating the former steps, depending on the person's input;
    - Mapping the perimetry of the person based on the input recorded.

**15.** A method for testing the perimetry of a person to detect a glaucoma comprising the following steps:

- Use of the distance between the center of the focus dot of light (F) and the center of the blind spot (200) located by the method of any one of claims 1 to 9 to calibrate the positions of testing dots of light;
- Displaying a focus dot of light (F) on a display;
- Displaying a testing dot of light at a certain position on the display and recording the input of the person for the displayed dot of light;
- Repeating the former step a plurality of times, whereby all testing dots of light differ from each other with regard to their position on the display, and whereby each position of a dot of light has to be tested with different brightnesses of the dot of light on the display;
- Displaying a reference dot of light within the area of the located blind spot of the person on the display and optionally repeating the former steps, depending on the person's input;
- Mapping the perimetry, being dependent on the brightness of the testing dots of lights, of the person based on the input recorded.

F

1, 11          10

Fig. 1

Fig. 2

Fig. 3

F

200

Fig. 4

Fig. 5

Fig. 6

Fig. 7

TP2LE

F

10, 200

Fig. 8

**Europäisches Patentamt**
**European Patent Office**
**Office européen des brevets**

# EUROPEAN SEARCH REPORT

**Application Number**

EP 24 17 5502

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 035 500 A (RORABAUGH DALE A [US] ET AL) 30 July 1991 (1991-07-30) <br> * column 9, line 5 - line 15 * <br> * column 15, line 6 - line 38 * <br> * column 16, line 14 - column 18, line 10 * <br> * column 20, line 1 - line 11 * <br> * column 21, line 3 - line 18 * <br> * column 24, line 39 - line 55 * <br> * column 38, line 51 - column 39, line 16 * <br> ----- | 1-9 | INV. <br> A61B3/024 <br> A61B3/032 <br> A61B3/103 |
| A | CN 113 116 287 A (ZHONGSHAN OPHTHALMIC CT SUN YAT SEN UNIV) 16 July 2021 (2021-07-16) <br> * paragraph [0059] - paragraph [0084] * <br> ----- | 1-15 | |
| X | US 2019/298166 A1 (SMITH AARON HENRY [US] ET AL) 3 October 2019 (2019-10-03) <br> * paragraph [0044] - paragraph [0049] * <br> * paragraph [0060] - paragraph [0065] * <br> * paragraph [0068] - paragraph [0074] * <br> * paragraph [0079] - paragraph [0080] * <br> * paragraph [0111] - paragraph [0112] * <br> ----- | 1 | |

**TECHNICAL FIELDS SEARCHED (IPC)**

A61B

The present search report has been drawn up for all claims

| Place of search | Date of completion of the search | Examiner |
|---|---|---|
| Munich | 28 January 2025 | Horváth, László |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

Europäisches
Patentamt

European
Patent Office

Office européen
des brevets

**Application Number**

EP 24 17 5502

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

22

Europäisches
Patentamt
European
Patent Office
Office européen
des brevets

**LACK OF UNITY OF INVENTION**
**SHEET B**

**Application Number**

EP 24 17 5502

The Search Division considers that the present European patent application does not comply with the requirements of unity of invention and relates to several inventions or groups of inventions, namely:

1. claims: 1-9

   A method for locating the blind spot in the visual field of a person comprising the following steps: a) Displaying a focus dot of light (F) that is fixated by one of the person's eyes on a display at a testing distance, whereby the focus dot of light (F) is preferably displayed in the central area of the display; b) Displaying a movable testing dot of light on the display, whereby the testing dot of light is moving straight from a starting point outside an expected blind spot along an axis towards the center (C) of said expected blind spot, c) Recording the end position of the testing dot of light that is taken when the person signals that it cannot see the testing dot of light on the display any more; d) Repeating steps b) and c) a plurality of times, whereby the testing dots of light vary independently from each other with respect to their starting point; and e) Mapping the located blind spot on basis of the end positions of the testing dots of light.
   ---

2. claims: 10-15

   A method for calibrating an ophthalmological test by using the blind spot located by the method of any one of claims 1 to 9 comprising the following steps: i) calculating the distance between the focus dot of light (F) and the located blind spot in pixels, and defining the distance between the focus dot of light (F) and the blind spot in degrees; ii) using the distance calculated in step i) for calibrating all positions or image sizes tested in the ophthalmological test.
   ---

## ANNEX TO THE EUROPEAN SEARCH REPORT
## ON EUROPEAN PATENT APPLICATION NO.

EP 24 17 5502

This annex lists the patent family members relating to the patent documents cited in the above-mentioned European search report.
The members are as contained in the European Patent Office EDP file on
The European Patent Office is in no way liable for these particulars which are merely given for the purpose of information.

28-01-2025

| Patent document cited in search report | | Publication date | Patent family member(s) | Publication date |
|---|---|---|---|---|
| US 5035500 | A | 30-07-1991 | NONE | |
| CN 113116287 | A | 16-07-2021 | NONE | |
| US 2019298166 | A1 | 03-10-2019 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050128434 A

- US 20220192482 A

**Non-patent literature cited in the description**

- **E. A. LOWRY et al.** Comparison of Peristat Online perimetry with the Humphrey Perimetry in a Clinic-Based Setting. *Translational Vision Science & Technology*, 2016, vol. 5 (4)

- **EY WONG** ; **JE KEEFFE** ; **JL RAIT et al.** Detection of undiagnosed glaucoma by eye health professionals. *Ophthalmology*, 2004, vol. 11 (1), 1508-1514